# EUROPEAN PATENT SPECIFICATION

(11) **EP 3 145 383 B1**
(45) Date of publication and mention of the grant of the patent: **24.03.2021**
(21) Application number: 14892299.0
(22) Date of filing: 23.05.2014
(51) Int. Cl.: A61B 1/00, A61B 1/05, A61B 1/313, H04N 5/225, H04N 13/207, H04N 13/296, H04N 13/218

(54) **3D LAPAROSCOPIC IMAGE CAPTURE APPARATUS WITH A SINGLE IMAGE SENSOR**
VORRICHTUNG ZUR ERFASSUNG LAPAROSKOPISCHER 3D-BILDER MIT EINEM EINZELNEN BILDSENSOR
APPAREIL DE CAPTURE D'IMAGE LAPAROSCOPIQUE 3D AVEC CAPTEUR D'IMAGE UNIQUE

(43) Date of publication of application: 29.03.2017
(73) Proprietor: Covidien LP, Mansfield, MA 02048 (US)
(72) Inventor: WU, Xinmin, Chengdu 611731 (CN); TAN, Wei, Shanghai 201318 (CN); LUO, Zhongchi, Shanghai 200125 (CN); SUN, Ruoxi, Shanghai 201112 (CN)
(74) Representative: Maschio, Antonio
(86) International application number: PCT/CN2014/078241
(87) International publication number: WO 2015/176298

(56) References cited:
- WO-A1-2007/097539
- WO-A1-2010/069169
- WO-A1-2013/025530
- CN-A- 1 912 664
- CN-A- 102 256 151
- CN-A- 103 070 660
- CN-A- 103 070 660
- CN-A- 103 841 404
- US-A1- 2003 174 237
- US-A1- 2004 138 527
- US-A1- 2013 250 061

## Description

### Technical Field

The present disclosure relates to an image capture apparatus and, more particularly, but not exclusively, to systems and methods for using the image capture apparatus with a single image sensor to reconstruct three-dimensional objects.

### BACKGROUND

Presently, the common method for capturing a 3D image is performed by using a stereo camera having two lenses. The stereo camera's two lenses have the same specifications, and a distance between the two lenses being about 7.7 cm, thus simulating an actual distance between a person's eyes. Parameters of the two lenses, such as focal lengths, apertures, and shutters are controlled by a processor. By triggering through a shutter release, images of the same area, but of different perspectives, are captured and used for simulating a left-eye image and a right-eye image of a person.

Specifically, the left-eye image and the right-eye image are respectively captured by the two lenses of the stereo camera. Since the two images captured by the stereo camera are be slightly different in angles, the 3D stereoscopic display can generate the depth of field based on the difference and combine the two images to display a 3D image. As long as capturing parameters are adjusted to be consistent with each other, a 3D image with a good imaging effect can be captured. However, in the structure of this type of stereo camera, two groups of lenses and sensors are required, and thus the cost is high. Another method for capturing a 3D image is to capture the image by rotating a single lens camera. However, an issue in this method is that the disparities of the near object and the far object between the two images may appear different from the real 3D image.

Thus, what improves the viewing of a 3D image is the ability to process the captured data/information/images/signals in new ways by presenting new image capture system configurations.

WO 2010/069169 discloses a single-sensor juxtaposing-type stereoscopic-image shooting method involves processing and storing image signals. Light from a subject is focalized via two batteries of optical imaging lenses with an interaxial distance of 57∼65 mm, producing left and right optical signals. The groups of optical signals are reflected by the first reflection mirror and the second reflection mirror, respectively. Each of the signals is rotated 90 degrees by a light path direction conversion component. Two sets of optical-signal images are produced in a CCD/CMOS sensor that is set to a 4:3 width-to-height ratio with a rotation setting of 90 degrees.

WO 2013/025530 discloses a minimally invasive surgical system including an image capture unit that includes a lens assembly and a sensor assembly. Sensor assembly includes a prism assembly and coplanar image capture sensors. Each of the coplanar image capture sensors has a common front end optical structure, e.g., lens assembly. A controller enhances images acquired by the coplanar image capture sensors. The enhanced images may include (a) visible images with enhanced feature definition; (b) images having increased image apparent resolution; (c) images having increased dynamic range; (d) images displayed in a way based on a pixel color component vector having three or more color components; and (e) images having extended depth of field.

### SUMMARY

The present invention provides an image capture apparatus as defined in the appended claims.

In accordance with aspects of the present disclosure, an image capture apparatus is presented. The image capture apparatus includes a first lens configured to receive a first image signal, a second lens configured to receive a second image signal, a first prism configured to reflect the first image signal, and a second prism configured to reflect the second image signal. The image capture apparatus further includes a third prism positioned between the first and second prisms, the third prism configured to receive the first and second image signals from the first and second prisms, respectively. The image capture apparatus also includes a single image sensor configured to receive the first and second image signals from the third prism. The first image signal is projected onto a left side of the single image sensor and the second image signal is projected onto the right side of the single image sensor.

The first and second image signals projected onto different sides (parts) of the single image sensor are used to reconstruct a three-dimensional image of an object captured by the first and second lenses.

In accordance with another aspect of the present disclosure, the image capture apparatus is a camera and the single image sensor is a CMOS (complementary metal-oxide-semiconductor) image sensor or a CCD (charge-coupled device) image sensor.

In accordance with yet another aspect of the present disclosure, the first lens is separated from the second lens by a predetermined distance. Moreover, the first and second lenses are synchronized with each other.

In accordance with another aspect of the present disclosure, the image capture apparatus is used during a surgical procedure.

Further scope of applicability of the present disclosure will become apparent from the detailed description given hereinafter. However, it should be understood that the detailed description and specific examples, while indicating illustrative embodiments of the present disclosure, are given by way of illustration only, since various changes and modifications within the spirit and scope of the present disclosure will become apparent to those skilled in the art from this detailed description.

### BRIEF DESCRIPTION OF THE DRAWINGS

The present disclosure and its various aspects and features are described hereinbelow with reference to the accompanying drawings, wherein:
FIG. 1 is a perspective view of an optical system including a pair of mirrors and a pair of prisms, which is provided by way of a general example, but does not form part of the claimed invention;
FIG. 2 is a perspective view of an optical system including three prisms in a series configuration, in accordance with an aspect of the present disclosure;
FIG. 3 illustrates a single image sensor separated into two portions, each portion configured to receive separate image signals, in accordance with an aspect of the present disclosure;
FIGS. 4A-4B illustrate at least one processor and at least one memory communicating with the single image sensor, in accordance with an aspect of the present disclosure;
FIG. 5 is a flowchart describing a method of reconstructing a three-dimensional object and is provided by way of a general example, but does not form part of the claimed invention;
FIG. 6 is a flowchart describing another method of reconstructing a three-dimensional object; and
FIG. 7 is a perspective view of a surgical positioning system including an instrument or medical device and an image capture apparatus having the optical system of FIG. 2, in accordance with an aspect of the present disclosure.

The figures depict embodiments of the present disclosure for purposes of illustration only. One skilled in the art will readily recognize from the following disclosure that alternative embodiments of the structures and methods illustrated herein may be employed without departing from the principles of the present disclosure described herein.

### DETAILED DESCRIPTION

Devices, systems, and methods for reconstructing 3D objects by using a camera having two lenses and a single image sensor are provided in accordance with the present disclosure and described in detailed below. The two separate camera lenses allow for the projection of two separate images onto a single image sensor. The two separate images pass through mirror and prism configurations before being received by the single image sensor.

Although the present disclosure will be described in terms of specific embodiments, it will be readily apparent to those skilled in this art that various modifications, rearrangements and substitutions may be made without departing from the spirit of the present disclosure. The scope of the present disclosure is defined by the claims appended hereto.

For the purposes of promoting an understanding of the principles of the present disclosure, reference will now be made to the exemplary embodiments illustrated in the drawings, and specific language will be used to describe the same. It will nevertheless be understood that no limitation of the scope of the present disclosure is thereby intended. Any alterations and further modifications of the inventive features illustrated herein, and any additional applications of the principles of the present disclosure as illustrated herein, which would occur to one skilled in the relevant art and having possession of this disclosure, are to be considered within the scope of the present disclosure.

The word "exemplary" is used herein to mean "serving as an example, instance, or illustration." Any embodiment described herein as "exemplary" is not necessarily to be construed as preferred or advantageous over other embodiments. The word "example" may be used interchangeably with the term "exemplary."

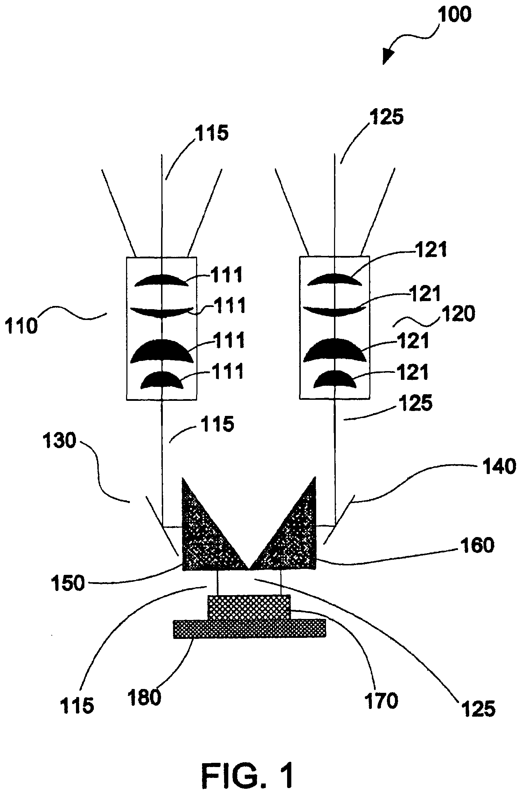

Referring to FIG. 1, a perspective view of an optical system including a pair of mirrors and a pair of prisms is provided by way of a general example, but this optical system does not form part of the claimed invention. The optical system 100 includes a first lens 110 and a second lens 120. The first lens 110 may be referred to as the left lens, whereas the second lens 120 may be referred to as the right lens. The first lens 110 may include a plurality of optical elements 111 and the second lens 120 may include a plurality of optical elements 121.

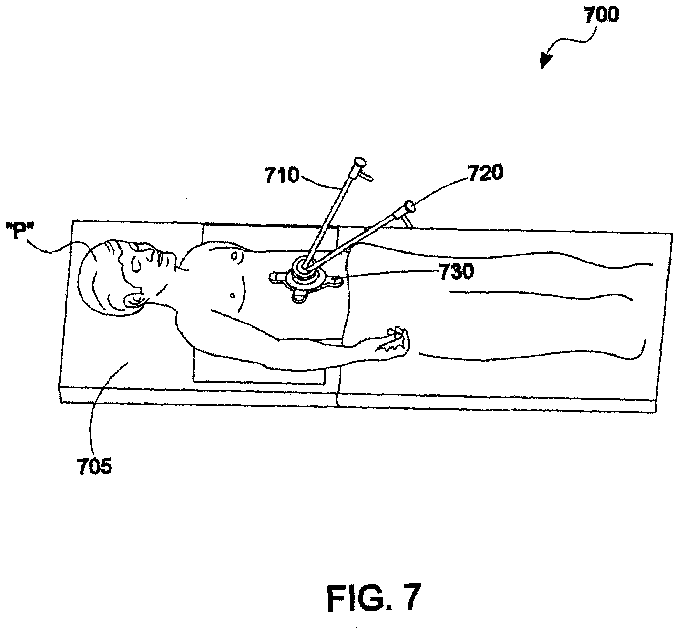

The first lens 110 is configured to receive a first image signal 115 that passes therethrough, whereas the second lens 120 is configured to receive a second image signal 125 that passes therethrough. The image signals 115 and 125 are light reflected off a surface of which an image is desired. In practice, the optical system 100 may be employed as part of a laparoscopic or endoscopic instrument (see FIG. 7) and the image signals 115 and 125 as light which originally emanated from the instrument (e.g., via a fiber optic strand) and is reflected back to the first and second lens's 110, 120. The first image signal 115 and the second image signal 125 may be images or light or other types of information/data captured by an image capturing apparatus, such as a camera.

The first image signal 115 is received by a first mirror 130, whereas the second image signal 125 is received by a second mirror 140. The first mirror 130 reflects the first image signal 115 to a first prism 150, whereas the second mirror 140 reflects the second image signal 125 to a second prism 160. The first and second prisms 150, 160 are positioned adjacent to each other. The first and second prisms 150, 160 may be in contact with each other. The first and second prisms 150, 160 may be triangular in shape. However, one skilled in the art may contemplate any shape or size for prisms 150, 160.

It is contemplated that first mirror 130 is co-axial with the first lens 110, whereas second mirror 140 is coaxial with the second lens 120. It is also contemplated that the first image signal 115 traverses substantially centrally through the first lens 110 such that the first image signal 115 is received on a central portion of first mirror 130. Similarly, it is also contemplated that the second image signal 125 traverses substantially centrally through the second lens 120 such that the second image signal 125 is received on a central portion of second mirror 140. It is also contemplated that the first and second prisms 150, 160 are off-centered or offset with respect to the first and second lenses 110, 120. Stated differently, the first and second prisms 150, 160 may be positioned at a midway point defined between the first and second lenses 110, 120.

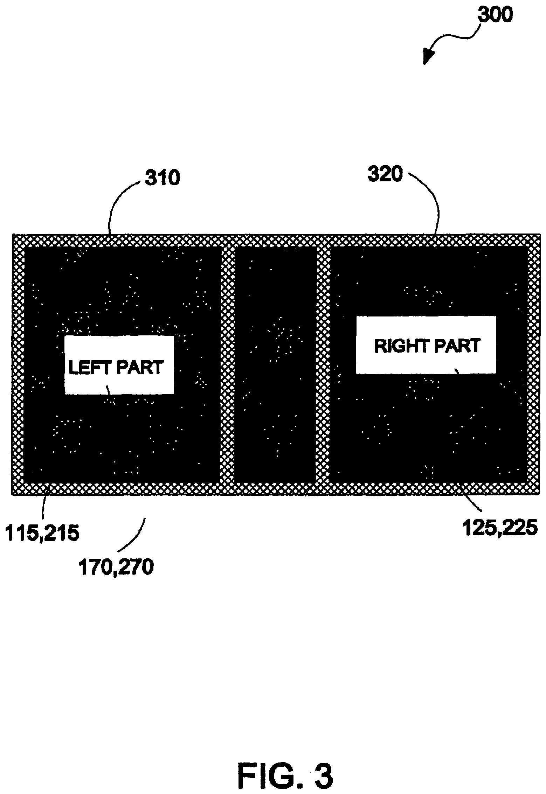

The first image signal 115 passes through the first prism 150 and is received by a single image sensor 170. Similarly, the second image signal 125 passes through the second prism 160 and is received by the single image sensor 170. The first image signal 115 is received on the left side of the single image sensor 170, whereas the second image signal 125 is received on the right side of the single image sensor 170. This is further illustrated in FIG. 3, described below. The single image sensor 170 may be in electrical communication with a printed circuit board (PCB) 180.

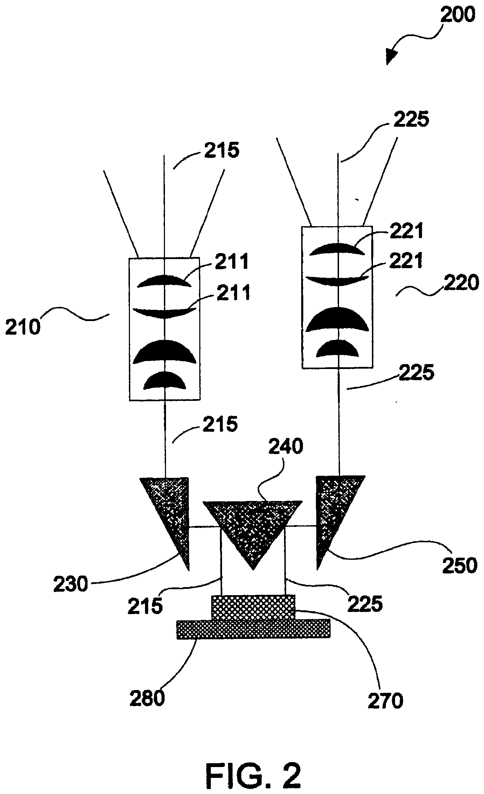

Referring to FIG. 2, a perspective view of an optical system including three prisms in a series configuration, in accordance with an aspect of the present disclosure is presented.

The optical system 200 includes a first lens 210 and a second lens 220. The first lens 210 may be referred to as the left lens, whereas the second lens 220 may be referred to as the right lens. The first lens 210 may include a plurality of optical elements 211 and the second lens 220 may include a plurality of optical elements 221.

The first lens 210 is configured to receive a first image signal 215 that passes therethrough, whereas the second lens 220 is configured to receive a second image signal 225 that passes therethrough. The first image signal 215 and the second image signal 225 may be images or light or other types of information/data captured by an image capturing apparatus, such as a camera.

The first image signal 215 is received by a first prism 230, whereas the second image signal 225 is received by a second prism 250. The prism 230 reflects the first image signal 215 to a third prism 240, and the second prism 250 reflects the second image signal 225 to the third prism 240. The first, second, and third prisms 230, 250, 240 are positioned adjacent to each other in a successive manner. It is contemplated that the three prisms 230, 250, 240 do not contact or abut each other. The third prism 240 is configured to be positioned between the first and second prisms 230, 250. The first, second, and third prisms 230, 250, 240 may be triangular in shape. However, one skilled in the art may contemplate any shape or size for prisms 230, 250, 240. The first and second prisms 230, 250 may be, for example, right triangles, whereas the third prism 240 may be, for example, an equilateral triangle.

It is contemplated that first prism 230 is co-axial with the first lens 210, whereas second prism 250 is coaxial with the second lens 220. It is also contemplated that the first image signal 215 traverses substantially centrally through the first lens 210 such that the first image signal 215 is received through a central portion of one side of the first prism 230. Similarly, it is also contemplated that the second image signal 225 traverses substantially centrally through the second lens 240 such that the second image signal 225 is received through a central portion of one side of the second prism 250. It is also contemplated that the third prism 240 is off-centered or offset with respect to the first and second lenses 210, 220. Stated differently, the third prism 240 may be positioned at a midway point defined between the first and second lenses 210, 220.

The first image signal 215 passes through the first prism 230 and is received by a single image sensor 270 via the third prism 240. Similarly, the second image signal 225 passes through the second prism 250 and is received by the single image sensor 270 via the third prism 240. The first image signal 215 is received on the left side of the single image sensor 270, whereas the second image signal 225 is received on the right side of the single image sensor 270. This is further illustrated in FIG. 3, described below. The single image sensor 270 may be in electrical communication with a printed circuit board (PCB) 280.

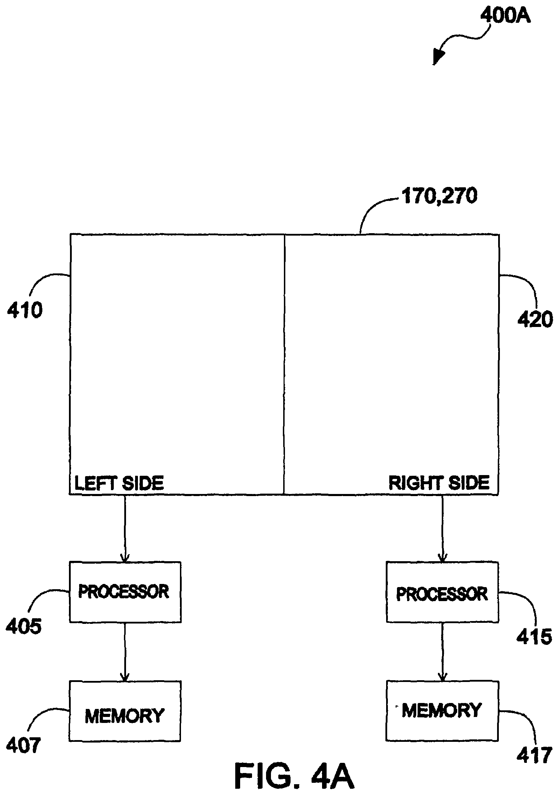

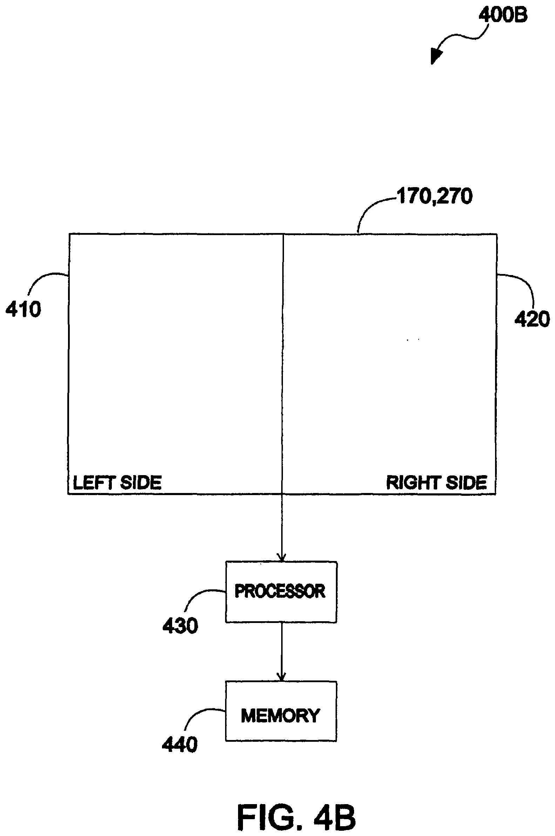

In summary, referring to both FIGS. 1 and 2, both the left and right lenses are used to capture an object within a scene. Both the left and right lenses are synchronized (on in-sync) with each other. The first and second lenses also have a fixed distance between them. Then with a mirror and prism configuration, or just a prism configuration, images captured by the left lens are passed therethrough to a left portion of the single image sensor, whereas images captured by the right lens are passed therethrough to a right portion of the single image sensor. Both the left images and the right images are simultaneously received by the single image sensor (without having left signals intersect with right signals). The left images and the right images are then processed by at least one processor (see FIGS. 4A, 4B, described below) and recorded to at least one memory device (see FIGS. 4A, 4B, described below). As a result, the distance between the lenses can vary in order to use such optical systems 100, 200 in a plurality of 3D image capture applications (e.g., laparoscopic systems used during surgical procedures).

Referring to FIG. 3, a single image sensor separated into two portions or segments, each portion or segment configured to receive separate image signals, in accordance with an aspect of the present disclosure is presented.

A top view 300 of the single image sensor 170, 270 is shown in FIG. 3. The image sensor 170, 270 includes a first area or region or portion or segment 310 and a second area or region or portion or segment 320. The first portion 310 may be referred to as the left part, whereas the second portion 320 may be referred to as the right part. The first image signals 115, 125 (see FIGS. 1 and 2) are received on the left part of the single image sensor 170, 270, respectively, whereas the second image signals 215, 225 (see FIGS. 1 and 2) are received on the right part of the single image sensor 170, 270, respectively.

Therefore, images picked up by the left lens are projected onto a left area of the single image sensor, whereas images picked up by the right lens are projected onto a right area of the single image sensor. The images remain separate as they progress through different mirror/prism configurations and eventually end up on separate areas of the single image sensor for separate processing and storage (see FIGS. 4A, 4B).

Thus, referring to FIGS. 1-3, the optical systems 100, 200 utilize two separate camera lenses to project two separate images onto a single image sensor (e.g., a CMOS - complementary metal-oxide-semiconductor) image sensor or a CCD (charge-coupled device) image sensor. Images or signals captured by the left lens (i.e., first lens 110, 210) are projected onto the left part 310 of the single image sensor 170, 270, respectively, whereas images or signals captured by the right lens (i.e., second lens 210, 220) are projected onto the right part 320 of the single image sensor 170, 270, respectively. Up to this point, the left images and the right images do not intersect or intermingle, but remain separate as they progress through the mirror/prisms configurations of FIGS. 1 and 2. Thereafter, the two images or signals are used to reconstruct a 3D image of the object in the scene. Since these images or signals are projected onto a single image sensor 170, 270, instead of projecting them onto two separate and distinct image sensors, image misrepresentation and optical distortion between the two images can be reduced, thus resulting in better color, brightness, and sharpness of the reconstructed 3D image.

In addition, as a result of the reduction in image misrepresentation and optical distortion, user side-effects when reconstructing 3D images captured by a traditional image capture apparatus, such as a camera, are also reduced. Further advantages include avoiding discordance of image representation between left and right images generated from two independent camera lenses, which also improves brightness, sharpness, color, and image quality of the reconstructed 3D image.

The devices and methods described herein are focused on the use of this disclosure in the medical context for capture and processing of visible spectrum light e.g., for use in generating of 3D laparoscopic and endoscopic images. However, the disclosure is not so limited and the technology may be employed in other spectra including, without limitation, autofluorescence imaging, Optical Coherence Tomography (OCT) imaging, and others, to generate 3D images. Additionally, the methods and systems described herein can be used for any type of 3D systems in any type of medical applications.

Further, while the optical systems 100, 200 may be employed as part of a new imaging device, e.g., a new laparoscope, the technology may also be employed with existing devices in which the optical system is employed either through a working channel of the laparoscope, or is connected to the exterior of the laparoscope or endoscope. Such an implementation has the advantage of providing both the traditional imaging clinicians are accustomed to and the enhanced 3D imaging of the present disclosure.

Referring to FIGS. 4A and 4B, at least one processor and at least one memory communicating with the single image sensor, in accordance with an aspect of the present disclosure are presented. FIG. 4A illustrates the single image sensor 170, 270 having a left side 410 electrically connected to a first processor 405 and a first memory 407. The single image sensor 170, 270 has a right side 420 electrically connected to a second processor 415 and a second memory 417. Thus, each side of the single image sensor 170, 270 may be connected to a different processor and different memory. Thus, a separate processor may be used for separately processing the first image on the left side and the second image on the right side of the single image sensor.

However, as illustrated in FIG. 4B, both the left side 410 and the right side 420 of the single image sensor 170, 270 may be connected to a common processor 430 and a common memory 440. One skilled in the art may contemplate a plurality of different configurations for processing and storing the left images, the right images, as well as the reconstructed 3D objects.

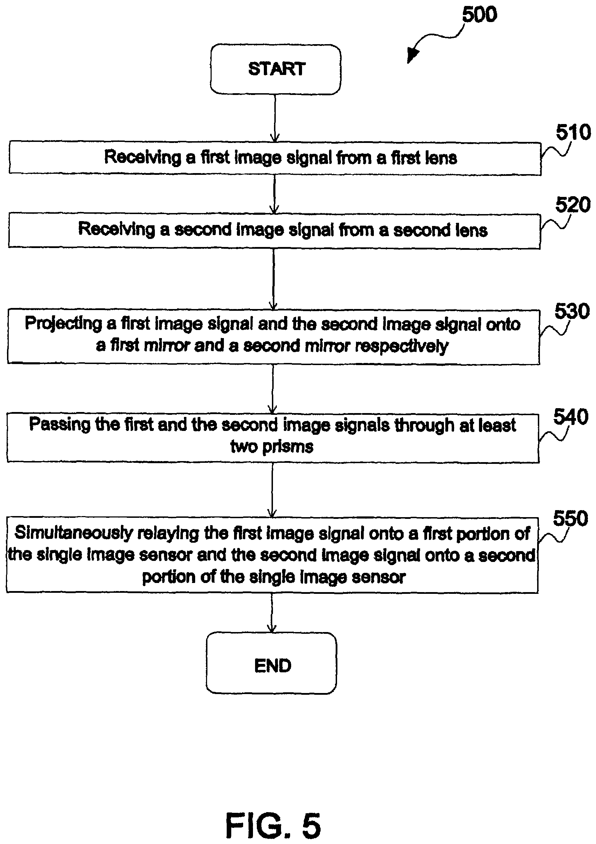

Referring to FIG. 5, a flowchart 500 illustrating a method of reconstructing a three-dimensional object is presented; this method is provided by way of a general example, but does not form part of the claimed invention.

The flowchart 500 includes the following steps. In step 510, a first image signal is received by the first lens of a camera. In step 520, a second image signal is received from a second lens of a camera. In step 530, the first image signal is projected onto a first mirror, whereas the second image signal is projected onto a second mirror. In step 540, the first image signal is passed through a first prism, whereas the second image signal is passed through a second prism. In step 550, the first image signal is relayed to a first part (or left portion) of a single image sensor and the second image signal is simultaneously relayed to a second part (right side) of the single image sensor. The process then ends. It is to be understood that the method steps described herein need not necessarily be performed in the order as described. Further, words such as "thereafter," "then," "next," etc. are not intended to limit the order of the steps. These words are simply used to guide the reader through the description of the method steps.

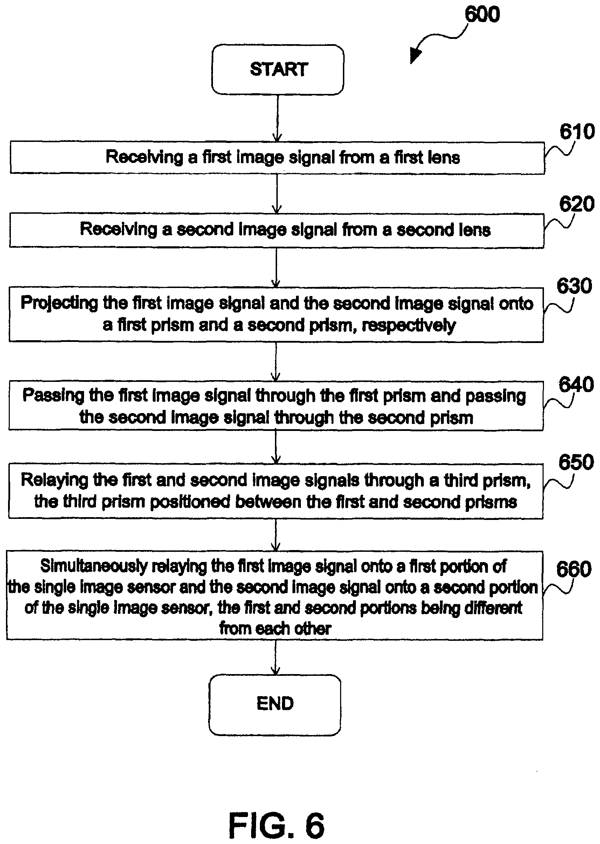

Referring to FIG. 6, a flowchart 600 illustrating another method of reconstructing a three-dimensional object.

The flowchart 600 includes the following steps. In step 610, a first image signal is received by the first lens of a camera. In step 620, a second image signal is received from a second lens of a camera. In step 630, the first image signal is projected onto a first prism, whereas the second image signal is projected onto a second prism. In step 640, the first image signal is passed through the first prism, whereas the second image signal is passed through the second prism. In step 650, the first and second image signals are relayed through a third prism, the third prism positioned between the first prism and the second prism. In step 660, the first image signal is relayed to a first part (or left portion) of a single image sensor and the second image signal is simultaneously relayed to a second part (right side) of the single image sensor. The process then ends. Further, the features and aspects of the present disclosure may be implemented in optical systems 100, 200 in any suitable fashion, e.g., via the hardware and software configuration of systems 100, 200 or using any other suitable software, firmware, and/or hardware.

Referring to FIG. 7, a perspective view of a surgical positioning system including an instrument or medical device and an image capture apparatus having either the optical system of FIG. 1 or the optical system of FIG. 2 is presented.

The surgical positioning system 700 is provided in accordance with the present disclosure and includes, an instrument fixation device 730 (optional), a surgical instrument 710, and an image capture apparatus 720. The surgical positioning system 700 is configured to position the surgical instrument 710 and the image capture apparatus 720 within a surgical site of the patient P lying on a surgical table 705. The image capture apparatus may include either the optical system 100 of FIG. 1 or the optical system 2 of FIG. 2 (described above). Therefore, the optical systems 100, 200 may be used with cameras utilized in a variety of surgical procedures.

For instance, when implemented via executable instructions, various elements of the present disclosure are in essence the code defining the operations of such various elements. The executable instructions or code may be obtained from a readable medium (e.g., a hard drive media, optical media, EPROM, EEPROM, tape media, cartridge media, flash memory, ROM, memory stick, and/or the like) or communicated via a data signal from a communication medium (e.g., the Internet). In fact, readable media may include any medium that may store or transfer information.

The computer means or computing means or processing means may be operatively associated with the assembly, and is directed by software to compare the first output signal with a first control image and the second output signal with a second control image. The software further directs the computer to produce diagnostic output. Further, a means for transmitting the diagnostic output to an operator of the verification device is included. Thus, many applications of the present disclosure could be formulated. The exemplary network disclosed herein may include any system for exchanging data or transacting business, such as the Internet, an intranet, an extranet, WAN (wide area network), LAN (local area network), satellite communications, and/or the like. It is noted that the network may be implemented as other types of networks.

Additionally, "code" as used herein, or "program" as used herein, may be any plurality of binary values or any executable, interpreted or compiled code which may be used by a computer or execution device to perform a task. This code or program may be written in any one of several known computer languages. A "computer," as used herein, may mean any device which stores, processes, routes, manipulates, or performs like operation on data. A "computer" may be incorporated within one or more transponder recognition and collection systems or servers to operate one or more processors to run the transponder recognition algorithms. Moreover, computer-executable instructions include, for example, instructions and data which cause a general purpose computer, special purpose computer, or special purpose processing device to perform a certain function or group of functions. Computer-executable instructions also include program modules that may be executed by computers in stand-alone or network environments. Generally, program modules include routines, programs, objects, components, and data structures, etc. that perform particular tasks or implement particular abstract data types.

Persons skilled in the art will understand that the devices and methods specifically described herein and illustrated in the accompanying drawings are non-limiting exemplary embodiments. The features illustrated or described in connection with one exemplary embodiment may be combined with the features of other embodiments.

## Claims

1. An image capture apparatus comprising:
a first lens (210) configured to receive a first image signal;
a second lens (220) configured to receive a second image signal;
a first prism (230) configured to reflect the first image signal;
a second prism (250) configured to reflect the second image signal;
a third prism (240) positioned between the first and second prisms, the third prism configured to receive the first and second image signals from the first and second prisms, respectively; and
a single image sensor (270) configured to receive the first and second image signals from the third prism, wherein the first image signal is projected onto the left side of the single image sensor and the second image signal is projected onto the right side of the single image sensor.

2. The image capture apparatus according to claim 1, wherein the first lens is separated from the second lens by a predetermined distance.

3. The image capture apparatus according to claim 1 or claim 2, wherein the first and second lenses are synchronized with each other.

4. The image capture apparatus according to claim 1 wherein the first and second image signals projected onto, respectively, the left side and the right side of the single image sensor are used to reconstruct a three-dimensional image of an object captured by the first and second lenses.

5. The image capture apparatus according to any preceding claim, wherein the image capture apparatus is a camera.

6. The image capture apparatus according to any preceding claim, wherein the single image sensor is a CMOS (complementary metal-oxide-semiconductor) image sensor or a CCD (charge-coupled device) image sensor.

7. The image capture apparatus according to any preceding claim, wherein the single image sensor electrically communicates with a printed circuit board (PCB).

8. The image capture apparatus according to any preceding claim, wherein the image capture apparatus is configured for use during a surgical procedure.

## Patentansprüche

1. Bilderfassungseinrichtung, die Folgendes umfasst:
eine erste Linse (210), die konfiguriert ist, um ein erstes Bildsignal zu empfangen;
eine zweite Linse (220), die konfiguriert ist, um ein zweites Bildsignal zu empfangen;
ein erstes Prisma (230), das konfiguriert ist, um das erste Bildsignal zu reflektieren;
ein zweites Prisma (250), das konfiguriert ist, um das zweite Bildsignal zu reflektieren;
ein drittes Prisma (240), das zwischen dem ersten und dem zweiten Prisma positioniert ist, wobei das dritte Prisma konfiguriert ist, um das erste und das zweite Bildsignal von dem ersten beziehungsweise dem zweiten Prisma zu empfangen; und
einen Einzelbildsensor (270), der konfiguriert ist, um das erste und das zweite Bildsignal von dem dritten Prisma zu empfangen, wobei das erste Bildsignal auf die linke Seite des Einzelbildsensors und das zweite Bildsignal auf die rechte Seite des Einzelbildsensors projiziert wird.

2. Bilderfassungseinrichtung nach Anspruch 1, wobei die erste Linse durch einen zuvor bestimmten Abstand von der zweiten Linse getrennt ist.

3. Bilderfassungseinrichtung nach Anspruch 1 oder 2, wobei die erste und die zweite Linse miteinander synchronisiert sind.

4. Bilderfassungseinrichtung nach Anspruch 1, wobei das erste und das zweite Bildsignal, das auf die linke Seite beziehungsweise die rechte Seite des Einzelbildsensors projiziert wird, verwendet werden, um ein dreidimensionales Bild eines Objekts zu rekonstruieren, das durch die erste und die zweite Linse erfasst wird.

5. Bilderfassungseinrichtung nach einem der vorhergehenden Ansprüche, wobei die Bilderfassungseinrichtung eine Kamera ist.

6. Bilderfassungseinrichtung nach einem der vorhergehenden Ansprüche, wobei der Einzelbildsensor ein CMOS(komplementärer Metalloxid-Halbleiter)-Bildsensor oder ein CCD(ladungsgekoppelte Vorrichtung)-Bildsensor ist.

7. Bilderfassungseinrichtung nach einem der vorhergehenden Ansprüche, wobei der Einzelbildsensor mit einer Leiterplatte (*printed circuit board* - PCB) elektrisch kommuniziert.

8. Bilderfassungseinrichtung nach einem der vorhergehenden Ansprüche, wobei die Bilderfassungseinrichtung zur Verwendung während eines chirurgischen Eingriffs konfiguriert ist.

## Revendications

1. Appareil de capture d'image comprenant :
une première lentille (210) configurée pour recevoir un premier signal d'image ;
une seconde lentille (220) configurée pour recevoir un second signal d'image ;
un premier prisme (230) configuré pour réfléchir le premier signal d'image ;
un deuxième prisme (250) configuré pour réfléchir le second signal d'image ;
un troisième prisme (240) positionné entre les premier et deuxième prismes, le troisième prisme étant configuré pour recevoir respectivement les premier et second signaux d'image des premier et deuxième prismes ; et
un capteur d'image unique (270) configuré pour recevoir les premier et second signaux d'image du troisième prisme, dans lequel le premier signal d'image est projeté sur le côté gauche du capteur d'image unique et le second signal d'image est projeté sur le côté droit du capteur d'image unique.

2. Appareil de capture d'image selon la revendication 1, dans lequel la première lentille est séparée de la seconde lentille par une distance prédéterminée.

3. Appareil de capture d'image selon la revendication 1 ou la revendication 2, dans lequel les première et seconde lentilles sont synchronisées l'une avec l'autre.

4. Appareil de capture d'image selon la revendication 1, dans lequel les premier et second signaux d'image projetés respectivement sur le côté gauche et le côté droit du capteur d'image unique sont utilisés pour reconstruire une image tridimensionnelle d'un objet capturé par les première et seconde lentilles.

5. Appareil de capture d'image selon l'une quelconque des revendications précédentes, dans lequel l'appareil de capture d'image est un appareil photographique.

6. Appareil de capture d'image selon l'une quelconque des revendications précédentes, dans lequel le capteur d'image unique est un capteur d'image CMOS (semi-conducteur à oxyde de métal complémentaire) ou un capteur d'image CCD (dispositif à couplage de charge).

7. Appareil de capture d'image selon l'une quelconque des revendications précédentes, dans lequel le capteur d'image unique communique électriquement avec une carte de circuit imprimé (PCB).

8. Appareil de capture d'image selon l'une quelconque des revendications précédentes, dans lequel l'appareil de capture d'image est conçu pour être utilisé pendant une intervention chirurgicale.
